Europäisches Patentamt

European Patent Office

Office européen des brevets

(19)

(11) Veröffentlichungsnummer: **0 158 928**
**B1**

(12)

# EUROPÄISCHE PATENTSCHRIFT

(45) Veröffentlichungstag der Patentschrift:
01.04.87

(21) Anmeldenummer: **85104035.2**

(22) Anmeldetag: **03.04.85**

(51) Int. Cl.⁴: **C 07 C 121/52,** C 07 C 120/14

(54) **Verfahren zur Herstellung von reinem 2,6-Dichlorbenzonitril.**

(30) Priorität: **06.04.84 DE 3412937**

(43) Veröffentlichungstag der Anmeldung:
**23.10.85 Patentblatt 85/43**

(45) Bekanntmachung des Hinweises auf die Patenterteilung:
**01.04.87 Patentblatt 87/14**

(84) Benannte Vertragsstaaten:
**BE CH DE FR GB IT LI NL**

(56) Entgegenhaltungen:
**DE - A - 2 029 118**
**GB - A - 947 167**
**US - A - 2 828 325**

(73) Patentinhaber: **SKW Trostberg Aktiengesellschaft,**
**Dr.-Albert-Frank-Strasse 32 Postfach 1150/1160,**
**D-8223 Trostberg (DE)**

(72) Erfinder: **Bayerl, Herbert, Jägerstrasse 8,**
**D-8223 Trostberg (DE)**
Erfinder: **Pollwein, Walter, Münchener Strasse 2,**
**D-8260 Mühldorf (DE)**

(74) Vertreter: **Weickmann, Heinrich, Dipl.-Ing. et al,**
**Patentanwälte Dipl.-Ing. H.Weickmann Dipl.-Phys.Dr.**
**K.Fincke Dipl.-Ing. F.A.Weickmann Dipl.-Chem. B. Huber**
**Dr.-Ing. H. Liska Dipl.-Phys.Dr. J. Prechtel**
**Postfach 860820, D-8000 München 86 (DE)**

## Beschreibung

Die vorliegende Erfindung betrifft ein Verfahren zur Herstellung von reinem 2,6-Dichlorbenzonitril durch Ammonoxidation von 2,6-Dichlortoluol.

2,6-Dichlorbenzonitril ist wegen seiner guten herbiziden Wirkung von grosser technischer Bedeutung (vgl. R. Wegler «Chemie des Pflanzenschutz- und Schädlingsbekämpfungsmittel» Bd. 5, S. 229-230, Springer-Verlag 1977).

Die Herstellung dieser Verbindung ist relativ kompliziert, da sie nur über Mehrstufenreaktionen zugänglich ist.

So wird beispielsweise in der DE-A 12 06 422 ein Verfahren zur Herstellung von 2,6-Dichlorbenzonitril beschrieben, wobei man 6-Chlor-2-nitrotoluol in Gegenwart eines heterocyclischen tertiären Amins mit Chlor umsetzt. Ganz abgesehen davon, dass 6-Chlor-2-nitrotoluol eine nur schwer zugängliche Ausgangssubstanz darstellt, besitzt dieses Verfahren gravierende Nachteile bezüglich Ausbeute und Reinheit. Das 2,6-Dichlorbenzonitril fällt nämlich nur als Nebenprodukt an, welches von dem Hauptprodukt 2,6-Dichlorbenzalchlorid sowie vom 2,6-Dichlrobenzylchlorid abgetrennt werden muss. Es ist deshalb verständlich, weshalb die Reinigung sehr hohe Investitions- und Betriebskosten erforderlich macht, wenn man zu einem halbwegs brauchbaren Produkt gelangen will.

Aus der DE-A 11 16 209 ist u.a. ebenfalls ein Verfahren zur Herstellung von 2,6-Dichlorbenzonitril bekannt, bei welchem dieses Produkt durch Ammonoxidation von 2,6-Dichlortoluol im Festbettverfahren in Ausbeuten von 27 bis 62% erhalten wird. Neben diesen nur mässigen Ausbeuten besteht ein weiterer Nachteil des Verfahrens darin, dass die Reinigung des gewünschten Produkts durch Destillation oder Kristallisation aus Lösungsmitteln erfolgt, wodurch das Verfahren relativ aufwendig wird.

Der vorliegenden Erfindung lag daher die Aufgabe zugrunde, ein Verfahren zur Herstellung von reinem 2,6-Dichlorbenzonitril durch Ammonoxidation von 2,6-Dichlortoluol an einem Katalysator auf Basis von Vanadin-Molybdänoxid zu entwickeln, welches die genannten Nachteile des Standes der Technik nicht aufweist, sondern es ohne besondere Aufarbeitungs- und Reinigungsschritte ermöglicht, ein sehr reines Produkt in guten Ausbeuten zu erhalten. Dabei soll vorzugsweise auch ein relativ unreines Ausgangsmaterial eingesetzt werden können.

Diese Aufgabe wurde erfindungsgemäss dadurch gelöst, dass man die Reaktion nach dem vorstehend angegebenen Verfahrensprinzip im Wirbelschichtverfahren durchführt und in die Reaktionsgase nach dem Verlassen des Wirbelschichtreaktors Wasser einsprüht. Es hat sich nämlich überraschenderweise gezeigt, dass man auf diese Weise ein sehr reines 2,6-Dichlorbenzonitril erhält, so dass ein weiterer Reinigungsschritt wie Destillation oder Umkristallisation nicht mehr erforderlich ist. Darüber hinaus fällt das Produkt hierbei besonders feinteilig an, was für bestimmte Anwendungen ebenfalls von Vorteil ist.

Beim erfindungsgemässen Verfahren wird – wie bei Ammonoxidationsverfahren üblich – ein Gasgemisch aus 2,6-Dichlortoluol, Ammoniak und Luft, welches mit Stickstoff verdünnt wird, durch ein Wirbelbett des Katalysators geschickt. Der Katalysator besteht vorzugsweise aus Vanadin-Molybdänoxid auf einem Aluminiumoxid-Träger wie er beispielsweise gemäss der DE-PS 10 63 144 hergestellt wird.

Nach Verlassen des Reaktors wird gemäss der vorliegenden Erfindung Wasser in die Reaktionsgase eingesprüht, wodurch eine optimale Trennung des Produkts von seinen Verunreinigungen erzielt wird. Diese Massnahme ist entscheidend für den Erfolg der Erfindung.

Die Menge des eingesprühten Wassers sollte vorzugsweise so bemessen werden, dass die Reaktionsgase hierdurch auf eine Temperatur von 20 bis 30°C abgeschreckt werden, was bei üblichen Kühlwassertemperaturen einer Menge von 10 bis 50 l, vorzugsweise 20 bis 30 l Wasser pro 100 ml 2,6-Dichlortoluol entspricht. Es können auch grössere Mengen an Wasser in das gasförmige Reaktionsgemisch eingesprüht werden, doch führt dies zu keiner nennenswerten weiteren Verbesserung der Produktqualität wie Reinheit oder Korngrösse. Verwendet man weniger als 10 l Wasser pro 10 ml 2,6-Dichlortoluol, so entsteht ein etwas grobkörnigeres, aber noch gut brauchbares Produkt, weil der Abschreckeffekt nicht so stark ausgeprägt ist. Das Einsprühen des Wassers kann mit den in der Technik üblichen Verfahren und Vorrichtungen durchgeführt werden. Durch das Einsprühen des Wassers wird erreicht, dass sich das 2,6-Dichlorbenzonitril als feinteiliger Feststoff abscheidet, während die Verunreinigungen wie z.B. Ammoniumchlorid oder Ammoniumcarbonat in der wässerigen Phase verbleiben. Das 2,6-Dichlorbenzonitril, welches in einer Reinheit von 98% anfällt, kann zusammen mit dem Wasser ausgeschleust werden und durch Filtration oder Zentrifugieren bequem von diesem abgetrennt werden, ohne dass ein weiterer Reinigungsschritt erforderlich ist. Falls notwendig kann das Produkt nach den üblichen Methoden getrocknet werden.

Das erfindungsgemässe Verfahren eignet sich auch hervorragend für eine kontinuierliche Durchführung, bei der das Wasser im Kreislauf geführt wird. Hierbei kann das Wasser nach der Abtrennung vom Reaktionsprodukt durch Wärmeaustauscher wieder auf die erforderliche Kühltemperatur gebracht und anschliessend nach Ausgleich eventueller Verluste wieder versprüht werden. Je nach Reaktionsdauer des Verfahrens empfiehlt es sich, einen Teil des verauchten Wassers durch Frischwasser zu ersetzen, um so eine möglichst konstante Produktqualität zu erreichen.

Das Molverhältnis von 2,6-Dichlortoluol zu Ammoniak beträgt zweckmässig 1:2 bis 5, vorzugsweise 1:2,5 bis 4, während sich ein molares Verhältnis von 2,6-Dichlortoluol zu Sauerstoff von 1:3 bis 5 als besonders vorteilhaft erwiesen hat.

Ein Molverhältnis ausserhalb dieses Bereichs ist jedoch möglich.

Die Reaktionstemperatur bei der Ammonoxidation sollte möglichst zwischen 400 und 500°C liegen. Bei wesentlicher Unterschreitung dieses Temperaturbereiches nimmt der Anteil an Verunreinigungen zu. Oberhalb von 500°C tritt in zunehmendem Masse Zersetzung ein, so dass die Ausbeute stark zurückgeht.

Die Kontaktzeit des 2,6-Dichlortoluols mit dem Katalysator beträgt je nach Höhe der Wirbelschicht und Gasgeschwindigkeit vorzugsweise 0,5 bis 1 Sekunde.

Das Verfahren der Erfindung zeichnet sich vor allem durch seinen geringen technischen Aufwand und den daraus resultierenden geringen Betriebs- und Investitionskosten aus sowie durch die hohe Reinheit des Endprodukts und der Möglichkeit, die Korngrösse des Produkts gezielt zu steuern. Ein weiterer Vorteil des Verfahrens besteht darin, dass man nicht unbedingt von einem sehr reinen 2,6-Dichlortoluol ausgehen muss. Man kann stattdessen auch ein ungereinigtes Ausgangsprodukt einsetzen, ohne dass sich dies auf die Reinheit des Endprodukts merklich auswirkt.

Die nachfolgenden Beispiele sollen die Erfindung näher erläutern.

*Beispiele*

Die nachfolgenden Beispiele 1-9 werden folgendermassen durchgeführt:

Ein Gasgemisch aus 2,6-Dichlortoluol, Ammoniak und Luft wird mit Stickstoff in den angegebenen Mengenverhältnissen (Bl = Betriebsliter bezogen auf 400°C und Normaldruck) durch das Katalysatorwirbelbett geschickt. Der Katalysator besteht aus einem Aluminiumoxidträger mit 17% Vanadinoxid und 7% Molybdänoxid. Nach einer durchschnittlichen Verweilzeit von 0,7-0,8 Sekunden verlassen die Reaktionsgase den Reaktor mit einer Temperatur von ca. 400°C. In dieses Reaktionsgemisch wird Wasser in den angegebenen Mengen eingesprüht und das Gemisch auf eine Temperatur von 24 bis 28°C abgekühlt. Das 2,6-Dichlorbenzonitril wird hierbei in sehr feiner Form abgeschieden und mit dem Wasser ausgetragen. Nach dem Abtrennen des Produkts durch Zentrifugieren wird das Wasser in einem Wärmetauscher abgekühlt und mittels einer Pumpe erneut zur Gaskühlung eingesetzt. Dem zurückgeführten Wasser werden die angegebenen Mengen an Frischwasser zugeführt, so dass die Verluste ausgeglichen werden und ein Teil des Kreislaufwassers ausgetauscht wird.

In den Beispielen 1-7 wird ein verunreinigtes 2,6-Dichlortoluol eingesetzt, welches 1,7% organische Verunreinigungen und 0,2% Wasser enthält und dunkelbraun gefärbt ist, während bei den Beispielen 8 und 9 ein sehr reines 2,6-Dichlortoluol verwendet wurde (0,1% Wasser; 0,1% sonstige Verunreinigungen).

*Beispiel 1:*
*Ausgangsverbindungen:*

| | |
|---|---|
| 2,6-Dichlortoluol (DCT) | 200 ml/h |
| Ammoniak ($NH_3$) | 158 Bl/h |
| Luft ($O_2$) | 940 Bl/h |
| Stickstoff ($N_2$) | 3.330 Bl/h |

*Molverhältnis:*

DCT : $NH_3$ : $O_2$ = 1:4:5

| | |
|---|---|
| Wasserumlauf | 40 l/h |
| Frischwasser | 100 ml/h |
| Abwasser | 150 ml/h |
| Ausbeute: 60% | |

*Produktreinheit (nach dem Trocknen):*

| | |
|---|---|
| 2,6-Dichlorbenzonitril | 99,7% |
| 2,6-Dichlortoluol | 0,1% |
| Wasser | 0,1% |
| Sonstiges Verunreinigungen | 0,1% |

*Beispiel 2:*

| | |
|---|---|
| 2,6-Dichlortoluol | 200 ml/h |
| Ammoniak | 119 Bl/h |
| Luft | 752 Bl/h |
| Stickstoff | 3.519 Bl/h |

*Molverhältnis:*

DCT : $NH_3$ : $O_2$ = 1:3:4
Wasserumlauf, Frisch- und Abwasser wie in Beispiel 1
Ausbeute: 63%

*Produktreinheit:*

| | |
|---|---|
| 2,6-Dichlorbenzonitril | 99,3% |
| 2,6-Dichlortoluol | 0,1% |
| Wasser | 0,1% |
| Sonstiges | 0,5% |

*Beispiel 3:*

| | |
|---|---|
| 2,6-Dichlortoluol | 200 ml/h |
| Ammoniak | 79 Bl/h |
| Luft | 564 Bl/h |
| Stickstoff | 3.729 Bl/h |

*Molverhältnis:*

DCT : $NH_3$ : $O_2$ = 1:2:3
Wasserumlauf, Frisch- und Abwasser wie in Beispiel 1
Ausbeute: 70%

*Produktreinheit:*

| | |
|---|---|
| 2,6-Dichlorbenzonitril | 98,2% |
| 2,6-Dichlortoluol | 0,3% |
| Wasser | 0,1% |
| Sonstiges | 1,4% |

*Beispiel 4:*

| | |
|---|---|
| 2,6-Dichlortoluol | 200 ml/h |
| Ammoniak | 99 Bl/h |
| Luft | 470 Bl/h |
| Stickstoff | 3.614 Bl/h |

*Molverhältnis:*

DCT : $NH_3$ : $O_2$ = 1:2,5:3,5
Wasserumlauf, Frisch- und Abwasser wie in Beispiel 1
Ausbeute: 72%

*Produktreinheit:*

| | |
|---|---|
| 2,6-Dichlorbenzonitril | 99,3% |
| 2,6-Dichlortoluol | 0,1% |
| Wasser | 0,1% |
| Sonstiges | 0,5% |

*Beispiel 5:*

Ausgangsverbindungen wie in Beispiel 4

| | |
|---|---|
| Wasserumlauf | 40 l/h |
| Frischwasser | 50 ml/h |
| Abwasser | 100 ml/h |

Ausbeute: 72%

*Produktreinheit:*

| | |
|---|---|
| 2,6-Dichlorbenzonitril | 99,8% |
| 2,6-Dichlortoluol | 0,2% |
| Wasser | 0,1% |
| Sonstiges | 0,9% |

*Beispiel 6:*

Ausgangsverbindungen wie in Beispiel 4

| | |
|---|---|
| Wasserumlauf | 40 l/h |
| Frischwasser | 150 ml/h |
| Abwasser | 200 ml/h |

Ausbeute: 70%

*Produktreinheit:*

| | |
|---|---|
| 2,6-Dichlorbenzonitril | 99,4% |
| 2,6-Dichlortoluol | 0,1% |
| Wasser | 0,1% |
| Sonstiges | 0,4% |

*Beispiel 7:*

Ausgangsverbindungen wie in Beispiel 4

| | |
|---|---|
| Wasserumlauf | 40 l/h |
| Frischwasser | 250 ml/h |
| Abwasser | 300 ml/h |

Ausbeute: 68%

*Produktreinheit:*

| | |
|---|---|
| 2,6-Dichlorbenzonitril | 99,5% |
| 2,6-Dichlortoluol | 0,1% |
| Wasser | 0,1% |
| Sonstiges | 0,3% |

*Beispiel 8:*

| | |
|---|---|
| 2,6-Dichlortoluol (Reinheit 99,8%) | 200 ml/h |
| Ammoniak | 101 Bl/h |
| Luft | 478 Bl/h |
| Stickstoff | 3.604 Bl/h |

*Molverhältnis:*

$DCT:NH_3:O_2 = 1:2,5:3,5$
Wasserumlauf, Frisch- und Abwasser wie in Beispiel 1
Ausbeute: 72%

*Produktreinheit:*

| | |
|---|---|
| 2,6-Dichlorbenzonitril | 99,4% |
| 2,6-Dichlortoluol | 0,1% |
| Wasser | 0,1% |
| Sonstiges | 0,4% |

*Beispiel 9:*

Ausgangsverbindungen wie in Beispiel 8

| | |
|---|---|
| Wasserumlauf | 40 l/h |
| Frischwasser | 250 ml/h |
| Abwasser | 30 ml/h |

Ausbeute: 70%

*Produktreinheit:*

| | |
|---|---|
| 2,6-Dichlorbenzonitril | 99,5% |
| 2,6-Dichlortoluol | 0,1% |
| Wasser | 0,1% |
| Sonstiges | 0,3% |

Das Produkt wies folgende Siebanalyse auf:

| | |
|---|---|
| < 31 μ | 15,9% |
| 31-125 μ | 18,6% |
| 125-500 μ | 45,3% |
| 500-710 μ | 15,5% |
| > 710 μ | 4,7% |

*Tabelle 1*

| Versuch | Edukt-Reinheit (% DCT)[1] | Molverhältnis $DCT:NH_3:O_2$ | Frischwasser (ml) | Ausbeute (%) | Produkt-Reinheit (% DCBN)[2] |
|---|---|---|---|---|---|
| 1 | 98,1 | 1:4:5 | 100 | 60 | 99,7 |
| 2 | 98,1 | 1:3:4 | 100 | 63 | 99,3 |
| 3 | 98,1 | 1:2:3 | 100 | 70 | 98,2 |
| 4 | 98,1 | 1:2,5:3,5 | 100 | 72 | 99,3 |
| 5 | 98,1 | 1:2,5:3,5 | 50 | 72 | 98,8 |
| 6 | 98,1 | 1:2,5:3,5 | 150 | 70 | 99,4 |
| 7 | 98,1 | 1:2,5:3,5 | 250 | 68 | 99,5 |
| 8 | 99,8 | 1:2,5:3,5 | 100 | 72 | 99,4 |
| 9 | 99,8 | 1:2,5:3,5 | 150 | 70 | 99,5 |

[1] DCT 2,6-Dichlortoluol
[2] DCBN 2,6-Dichlorbenzonitril

**Patentansprüche**

1. Verfahren zur Herstellung von reinem 2,6-Dichlorbenzonitril durch Ammonoxidation von 2,6-Dichlortoluol an einem Katalysator auf Basis von Vanadin-Moylbdänoxid, dadurch gekennzeichnet, dass man die Reaktion im Wirbelschichtverfahren durchführt und in die Reaktionsgase nach dem Verlassen des Wirbelbettreaktors Wasser einsprüht.

2. Verfahren nach Anspruch 1, dadurch gekennzeichnet, dass man Wasser in der Menge einsprüht, dass die Reaktionsgase auf eine Temperatur von 20 bis 30°C abgeschreckt werden.

3. Verfahren nach den Ansprüchen 1 und 2, dadurch gekennzeichnet, dass man Wasser in Mengen von 10 bis 50 l, insbesondere 20 bis 30 l, pro 100 ml 2,6-Dichlortoluol einsprüht.

4. Verfahren nach den Ansprüchen 1 bis 3, dadurch gekennzeichnet, dass das Molverhältnis von 2,6-Dichlortoluol zu Ammoniak 1:2 bis 5, vorzugsweise 1:2,5 bis 4 beträgt.

5. Verfahren nach den Ansprüchen 1 bis 4, dadurch gekennzeichnet, dass das Molverhältnis von 2,6-Dichlortoluol zu Sauerstoff 1:3 bis 5 beträgt.

6. Verfahren nach den Ansprüchen 1 bis 5, dadurch gekennzeichnet, dass die Reaktionstemperatur 400 bis 500°C beträgt.

7. Verfahren nach den Ansprüchen 1 bis 6, dadurch gekennzeichnet, dass die Kontaktzeit des 2,6-Dichlortoluols mit dem Katalysator 0,5 bis 1 Sekunde beträgt.

8. Verfahren nach den Ansprüchen 1 bis 7, dadurch gekennzeichnet, dass man es kontinuierlich durchführt.

## Claims

1. Process for the preparation of pure 2,6-dichlorobenzonitrile by the ammonoxidation of 2,6-dichlorotoluene on a catalyst based on vanadium-molybdenum oxide, characterised in that one carries out the reaction in a fluidised bed process and water is sprayed into the reaction gases after leaving the fluidised bed reactor.

2. Process according to claim 1, characterised in that one sprays in water in such an amount that the reaction gases are quenched to a temperature of 20 to 30°C.

3. Process according to claim 1 or 2, characterised in that one sprays in water in amounts of 10 to 50 l., especially of 20 to 30 l., per 100 ml. of 2,6-dichlorotoluene.

4. Process according to claims 1 to 3, characterised in that the mole ratio of 2,6-dichlorotoluene to ammonia amounts to 1:2 to 5, preferably 1:2.5 to 4.

5. Process according to claims 1 to 4, characterised in that the mole ratio of 2,6-dichlorotoluene to oxygen amounts to 1:3 to 5.

6. Process according to claims 1 to 5, characterised in that the reaction temperature amounts to 400 to 500°C.

7. Process according to claims 1 to 6, characterised in that the contact time of the 2,6-dichlorotoluene with the catalyst amounts to 0.5 to 1 second.

8. Process according to claims 1 to 7, characterised in that it is carried out continuously.

## Revendications

1. Procédé de préparation du 2,6-dichlorobenzonitrile pur par ammoxydation du 2,6-dichlorotoluène sur un catalyseur à base d'oxyde de vanadium-molybdène, caractérisé en ce qu'on effectue la réaction par le procédé en lit tourbillonnaire et on pulvérise de l'eau dans les gaz réactionnels après leur sortie du réacteur à lit tourbillonnaire.

2. Procédé suivant la revendication 1, caractérisé en ce qu'on pulvérise de l'eau dans une quantité telle que les gaz réactionnels soient refroidis brusquement à une température de 20 à 30°C.

3. Procédé suivant les revendications 1 à 2, caractérisé en ce qu'on pulvérise de l'eau à raison de 10 à 50 l, en particulier de 20 à 30 l pour 100 ml de 2,6-dichlorotoluène.

4. Procédé suivant les revendications 1 à 3, caractérisé en ce que le rapport molaire du 2,6-dichlorotoluène à l'ammoniac est de 1:2 à 5, de préférence de 1:2,5 à 4.

5. Procédé suivant les revendications 1 à 4, caractérisé en ce que le rapport molaire du 2,6-dichlorotoluène à l'oxygène est de 1:3 à 5.

6. Procédé suivant les revendications 1 à 5, caractérisé en ce que la température de réaction est de 400 à 500°C.

7. Procédé suivant les revendications 1 à 6, caractérisé en ce que le temps de contact du 2,6-dichlorotoluène avec le catalyseur est de 0,5 à 1 seconde.

8. Procédé suivant les revendications 1 à 7, caractérisé en ce qu'on l'effectue en continu.